# EUROPEAN PATENT SPECIFICATION

(11) **EP 4 510 909 B1**
(45) Date of publication and mention of the grant of the patent: **09.12.2026**
(21) Application number: 23720243.7
(22) Date of filing: 13.04.2023
(51) Int. Cl.: A61B 5/00, A61B 5/0215, A61B 5/027

(54) **FLEX CIRCUIT AROUND CORE WIRE IN INTRALUMINAL DEVICE**
FLEXIBLE SCHALTUNG UM EINEN KERNDRAHT IN EINER INTRALUMINALEN VORRICHTUNG
IRCUIT FLEXIBLE AUTOUR D'UN FIL CENTRAL DANS UN DISPOSITIF INTRALUMINAL

(30) Priority: 20.04.2022 US 202263332763 P
(43) Date of publication of application: 26.02.2025
(73) Proprietor: Koninklijke Philips N.V., 5656 AE Eindhoven (NL)
(72) Inventor: NAVARRO, Jose, 5656 AG Eindhoven (NL)
(74) Representative: Philips Intellectual Property & Standards
(86) International application number: PCT/EP2023/059602
(87) International publication number: WO 2023/202934

(56) References cited:
- WO-A1-2013/074036
- US-A- 6 090 052
- US-A1- 2002 040 185
- US-A1- 2019 133 527
- US-A1- 2020 324 081

## Description

### TECHNICAL FIELD

The subject matter described herein relates to intraluminal physiology sensing devices (e.g., an intravascular pressure sensing and/or flow sensing guidewire). For example, the intraluminal device may include a flex circuit for providing power and/or data communication between a distal sensor and a proximal electrical connector.

### BACKGROUND

Existing intravascular guidewires with a sensor have fine-gauge electrical wires that provide transmission of electrical signals for the sensor. Manufacture of such devices may include many steps that involve human operator or machine contact with the fine-gauge electrical wires. Because of their delicate nature, the fine-gauge electrical wires are prone to damage as a result of such contact. For example, the conductors themselves may break and the insulation may be damaged. This leads to poor or no electrical connectivity for the sensor. Additionally, the fine-gauge electrical wires typically have circular cross-section, which occupies space with the very small outer diameter of the guidewire.

A prior art intraluminal device which employs a flexible printed circuit board to connect to a sensor is known from US 6 090 052.

The information included in this Background section of the specification, including any references cited herein and any description or discussion thereof, is included for technical reference purposes only and is not to be regarded as subject matter by which the scope of the disclosure is to be bound.

### SUMMARY

A device in accordance with the invention is disclosed in claim 1.

Disclosed are intraluminal physiology sensing devices (e.g., an intravascular pressure-sensing and/or flow-sensing guidewire) that include flex circuits for power and signal communication. The flex circuit may be formed separately and then applied to one or components of an intraluminal physiology sensing device, which permits improved electrical/mechanical performance and reduce or eliminate damage associated with handling the fine-gauge electrical wires. Because flex circuits have a flatter profile, flex circuits allow for a larger outside diameter (OD) distal core wire and/or proximal core wire and eliminate damage/scrap due to multi-filar bundle handling, which may enable lower electrical resistance/impedance along the length of the electro-mechanical intraluminal sensing device. Furthermore, the straightness and torque response of the intraluminal device with flex circuits may be improved as well because ribbons and/or multi-filar conductor bundles add stiffness and local torques to a guidewire or catheter.

The flex circuit assembly disclosed herein has particular, but not exclusive, utility for intraluminal medical catheters, guidewires, or guide catheters.

In an exemplary aspect, an intraluminal device is provided. The intraluminal device includes a flexible elongate member configured to be positioned within a body lumen of a patient, wherein the flexible elongate member comprises: a distal portion comprising a distal core member and a sensor, wherein the distal core member comprises a length between a distal end and a proximal end; a proximal portion comprising a proximal electrical conductor; and a flex circuit positioned around a majority of the length of the distal core member, wherein the flex circuit comprises a distal portion coupled to the sensor and a proximal portion coupled to a proximal electrical conductor such that the proximal electrical conductor is in electrical communication with the sensor via the flex circuit.

In some aspects, the flex circuit comprises: a polymer base; and a conductive trace formed on the polymer base. In some aspects, the flex circuit further comprises a polymer topcoat formed over the conductive trace. In some aspects, the flex circuit further comprises: a distal conductive pad formed on the polymer base at a distal portion of the flex circuit; and a proximal conductive pad formed on the polymer base at a proximal portion of flex circuit, wherein the conductive trace is coupled to the distal conductive pad and the proximal conductive pad, wherein the proximal electrical conductor is coupled to the flex circuit at the proximal conductive pad, and wherein the sensor is coupled to the flex circuit at the distal conductive pad. In some aspects, an adhesive is positioned between the flex circuit and the distal core member, wherein the adhesive is configured to couple the flex circuit and the distal core member. In some aspects, flex circuit is wrapped in a spiral pattern around the distal core member. In some aspects, the flex circuit is rolled in a cylindrical configuration around the distal core member. In some aspects, the flex circuit extends parallel to a longitudinal axis of the flexible elongate member around the distal core member. In some aspects, the flex circuit is formed prior to being positioned around the distal core member. In some aspects, the flex circuit is formed as a sheet, wherein at least one conductive trace of the flex circuit is formed across the sheet, and wherein the sheet is wrapped around the distal core member. In some aspects, the proximal portion further comprises a proximal core member, wherein the proximal core member comprises a distal end and a proximal end, wherein the flex circuit is positioned around the proximal core member. In some aspects, the distal portion further comprises: a polymer coating positioned around the flex circuit. In some aspects, the polymer coating is in direct contact with the flex circuit. In some aspects, the distal portion further comprises: a hydrophilic coating positioned around the flex circuit. In some aspects, the hydrophilic coating is in direct contact with the flex circuit.

In an exemplary aspect, an apparatus is provided. The apparatus includes an intravascular guidewire configured to be positioned within a blood vessel of a patient, the intravascular guidewire comprising: a distal portion comprising a distal core member and a sensor, wherein the distal core member comprises a length between a distal end and a proximal end; a proximal portion comprising a proximal electrical conductor; and a flex circuit positioned around a majority of the length of the distal core member, wherein the flex circuit comprises a distal portion coupled to the sensor and a proximal portion coupled to a proximal electrical conductor such that the proximal electrical conductor is in electrical communication with the sensor via the flex circuit.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the metal ink conductor assembly, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
Fig. 1 is a diagrammatic top view of an intravascular device, according to aspects of the present disclosure.
Fig. 2 is a diagrammatic side view of an intravascular sensing system that includes an intravascular device, according to aspects of the present disclosure.
Fig. 3A is a schematic top view of an unrolled flex circuit for application to a portion of an intravascular device with electrical conductors/traces formed in the flex circuit, according to aspects of the present disclosure.
Fig. 3B is a schematic end view of a rolled flex circuit for application to a portion of the intravascular device from direction P shown in Fig. 3A rolled in a cylindrical configuration along a longitudinal axis and a cross-sectional end view along section line A-A in Fig. 5, according to aspects of the present disclosure.
Figs. 4A-4D are schematic side views of a portion of a flex circuit including electrical conductors/traces disposed onto a substrate of the flex circuit, according to aspects of the present disclosure.
Fig. 5 is a diagrammatic side view of a portion of an intraluminal device 102 with electrical conductors/traces 328 formed in the flex circuit 320, according to aspects of the present disclosure.
Fig. 6 is a diagrammatic cross-sectional side view of a portion of an intraluminal device 102 with electrical conductors/traces 328 formed in the flex circuit 320, according to aspects of the present disclosure.
Fig. 7 is a diagrammatic cross-sectional side view of a portion of an intraluminal device 102 with electrical conductors/traces 328 formed in the flex circuit 320, according to aspects of the present disclosure.

### DETAILED DESCRIPTION

Disclosed is a flex circuit assembly that provides an improved electrical/mechanical performance for an intraluminal sensing device and that eliminates manufacturing issues associated with conductive filars. Multi-filar bundles (e.g., bifilar or trifilar) are groupings or multiple (e.g., two or three) conductors, with each conductor being surrounded by insulating coating. Manufacturing or assembly processes that require tensioning and over-coating of a multi-filar bundle may benefit from the flex circuit assembly of the present disclosure.

Replacing embedded filars with flex circuits enables the use of conductive materials that may be formed separately and then applied to one or components of a flexible elongate member. Flex circuits allow for a larger outside diameter (OD) distal core wire and/or proximal core wire and eliminate damage/scrap due to multi-filar bundle handling. This may enable lower electrical resistance/impedance along the length of the electro-mechanical intraluminal sensing device. Because ribbons and/or multi-filar conductor bundles add stiffness and local torques to a guidewire or catheter, the straightness and torque response of the intraluminal device with flex circuits may be improved as well, which may lead to a reduction in mechanical "whipping" responses when the device is manipulated within intravascular anatomy. Whipping occurs when the distal portion of the guidewire does not smoothly rotate with the proximal portion of the guidewire (e.g., which is controlled by a user), instead the distal portion of the guidewire rapidly rotates inside the blood vessel to catch up to the rotational position of the proximal portion of the guidewire.

Other manufacturing issues may also be alleviated by using the flex circuit assembly of the present disclosure. For example, in a current manufacturing process, a proximal end of a distal core and a distal end of a proximal core are reduced in diameter so that the multi-filar bundle may fit inside of the hypotube and allow sufficient space for the multi-filar bundle. This reduction in diameter makes it more likely for this area of the wire to kink during bending/handling. By applying the flex circuits to the outside of the distal core wire and/or the proximal core wire, the flex circuit assembly reduces or eliminates these difficulties.

Example devices incorporating a multi-filar conductor bundle and/or conductive ribbons include intraluminal medical guidewire devices as described for example in U.S.

Patent No. 10,595,820 B2, U.S. Patent Publication Nos. 2014/0187874, 2016/0058977, and 2015/0273187, and in U.S. Provisional Patent Application No. 62/552,993 (filed August 31, 2017).

These descriptions are provided for exemplary purposes only and should not be considered to limit the scope of the metal ink conductor assembly. Certain features may be added, removed, or modified without departing from the spirit of the claimed subject matter.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. Further, while the embodiments of the present disclosure may be described with respect to a blood vessel, it will be understood that the devices, systems, and methods described herein may be configured for use in any suitable anatomical structure or body lumen including a blood vessel, blood vessel lumen, an esophagus, eustachian tube, urethra, fallopian tube, intestine, colon, and/or any other suitable anatomical structure or body lumen. In other embodiments, the devices, systems, and methods described herein may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood vessels, chambers or other parts of the heart, and/or other systems of the body. In addition to natural structures, the device may be used to examine man-made structures such as, but without limitation, heart valves, stents, shunts, filters, and other devices. For the sake of brevity, however, the numerous iterations of these combinations will not be described separately.

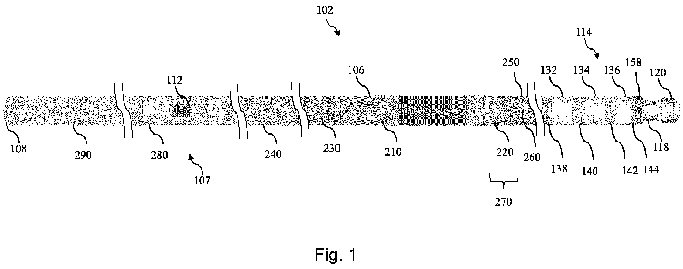

Fig. 1 is a diagrammatic top view of an intraluminal device 102, according to aspects of the present disclosure. The intraluminal device 102 may be an intravascular, intraluminal, or endoluminal device, such as a guidewire, a catheter, or a guide catheter sized and shaped for positioning within a blood vessel of a patient. The intraluminal device 102 includes a sensor 112. For example, the sensor 112 may be a pressure sensor configured to measure a pressure of blood flow within the vessel of the patient. The intraluminal device 102 includes the flexible elongate member 106. The sensor 112 is disposed at the distal portion 107, also referred to as a distal subassembly, of the flexible elongate member 106. The sensor 112 may be mounted at the distal portion 107 within a housing 280 in some embodiments. A flexible tip coil 290 extends between the housing 280 and the distal tip 108. The connection portion 114 is disposed at the proximal portion 109, also referred to as a proximal subassembly, of the flexible elongate member 106. The connection portion includes the conductive portions 132, 134, 136. In some embodiments, the conductive portions 132, 134, 136 may be conductive ink that is printed and/or deposited around the flexible elongate member 106. In some embodiments, the conductive portions 132, 134, 136 may be conductive, metallic rings that are positioned around the flexible elongate member. The locking section 118 and knob or retention section 120 are disposed at the proximal portion 109 of the flexible elongate member 106.

The intraluminal device 102 in Fig. 1 includes a distal core 210 and a proximal core 220. The distal core 210 and the proximal core 220 are metallic components forming part of the body of the intraluminal device 102. For example, the distal core 210 and the proximal core 220 are flexible metallic rods that provide structure for the flexible elongate member 106. The diameter of the distal core 210 and the proximal core 220 that electrically and mechanically couples the distal core 210 to the proximal core 220 may vary along its length. A joint between the distal core 210 and proximal core 220, which electrically and mechanically couples the distal core 210 to the proximal core 220, is surrounded and contained by a hypotube 215, which is a tubular member.

In some embodiments, the intraluminal device 102 includes a distal assembly and a proximal assembly that are electrically and mechanically joined together, which results in electrical communication between the sensor 112 and the conductive portions 132, 134, 136. For example, pressure data obtained by the sensor 112 (in this example, sensor 112 is a pressure sensor) may be transmitted to the conductive portions 132, 134, 136. Control signals from a computer in communication with the intraluminal device 102 may be transmitted to the sensor 112 via the conductive portions 132, 134, 136. The distal subassembly includes the distal core 210. The distal subassembly may also include the sensor 112, distal conductive members 230, and/or one or more layers of polymer/plastic 240 surrounding the distal conductive members 230 and the distal core 210. For example, the polymer/plastic layer(s) may protect the distal conductive members 230. The proximal subassembly includes the proximal core 220. The proximal subassembly may also include one or more layers of polymer layer(s) 250 (hereinafter polymer layer 250) surrounding the proximal core 220 and/or conductive ribbons 260 embedded within the one or more layers of polymer layer(s) 250. In some embodiments, the proximal subassembly and the distal subassembly may be separately manufactured. During the assembly process for the intraluminal device 102, the proximal subassembly and the distal subassembly may be electrically and mechanically joined together. As used herein, flexible elongate member may refer to one or more components along the entire length of the intraluminal device 102, one or more components of the proximal subassembly (e.g., including the proximal core 220, etc.), and/or one or more components the distal subassembly (e.g., including the distal core 210, etc.).

In various embodiments, the intraluminal device 102 may include one, two, three, or more core wires, also referred to as core members, extending along its length. For example, a single core wire may extend substantially along the entire length of the flexible elongate member 106. In such embodiments, the locking section 118 and the knob or retention section 120 may be integrally formed at the proximal portion of the single core wire. The sensor 112 may be secured at the distal portion of the single core wire. In other embodiments, such as the embodiment illustrated in Fig. 1, the locking section 118 and the knob or retention section 120 may be integrally formed at the proximal portion of the proximal core 220. The sensor 112 may be secured at the distal portion of the distal core 210. The intraluminal device 102 includes one or more distal conductive members 230 in communication with the sensor 112. For example, the distal conductive members 230 may be one or more electrical wires that are directly in communication with the sensor 112. In some instances, the distal conductive members 230 are electrically and mechanically coupled to and in electrical communication with the sensor 112 by, e.g., soldering. In some instances, the distal conductive members 230 include two or three electrical wires (e.g., a bifilar cable or a trifilar cable). An individual electrical wire may include a bare metallic conductor surrounded by one or more insulating layers. The distal conductive members 230 may extend along the length of the distal core 210. For example, at least a portion of the distal conductive members 230 may be spirally wrapped in a spiral pattern around the distal core 210.

The intraluminal device 102 includes one or more conductive ribbons 260 at the proximal portion of the flexible elongate member 106. The conductive ribbons 260 are embedded within polymer layer(s) 250. The conductive ribbons 260 are directly in communication with the conductive portions 132, 134, and/or 136. In some instances, the distal conductive members 230 are electrically and mechanically coupled to and in electrical communication with the sensor 112 by, e.g., soldering. In some instances, the conductive portions 132, 134, and/or 136 include conductive ink (e.g., metallic nano-ink, such as silver or gold nano-ink) that is deposited or printed directed over the conductive ribbons 260.

As described herein, electrical communication between the distal conductive members 230 and the conductive ribbons 260 may be established at the connection region 270 of the flexible elongate member 106. By establishing electrical communication between the distal conductive members 230 and the conductive ribbons 260, the conductive portions 132, 134, 136 may be in electrically communication with the sensor 112.

In some embodiments represented by Fig. 1, intraluminal device 102 includes the locking section 118 and the knob or retention section 120. To form the locking section 118, a machining process is necessary to remove the polymer layer 250 and the conductive ribbons 260 in the locking section 118 and to shape proximal core 220 in the locking section 118 to the desired shape. As shown in Fig. 1, the locking section 118 includes a reduced diameter while the knob or retention section 120 has a diameter substantially similar to that of proximal core 220 in the connection portion 114. In some instances, because the machining process removes conductive ribbons in locking section 118, proximal ends of the conductive ribbons 260 would be exposed to moisture and/or liquids, such as blood, saline solutions, disinfectants, and/or enzyme cleaner solutions, an insulation layer 158 is formed over the proximal end portion of the connection portion 114 to insulate the exposed conductive ribbons.

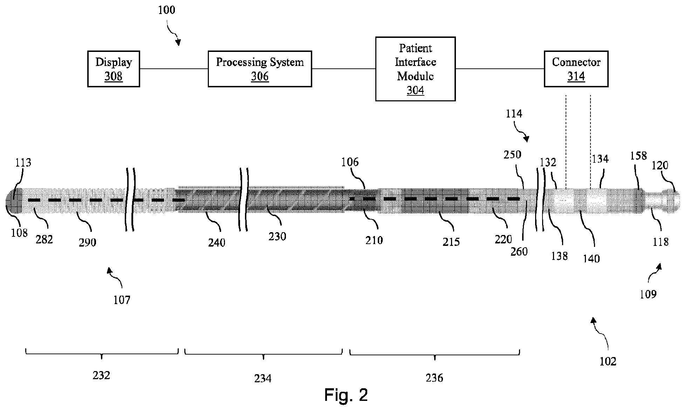

Fig. 2 is a diagrammatic side view of an intraluminal (e.g., intravascular) sensing system 100 that includes an intraluminal device 102 includes distal conductive members 230 (e.g., a multi-filar electrical conductor bundle) and conductive ribbons 260, according to aspects of the present disclosure. The intraluminal device 102 may be an intravascular guidewire sized and shaped for positioning within a blood vessel of a patient. The intraluminal device 102 includes a distal tip 108 and a sensor 113. For example, the sensor 113 may be a pressure sensor and/or flow sensor configured to measure a pressure of blood flow within the vessel of the patient, or another type of sensor including but not limited to a temperature or imaging sensor, or combination sensor measuring more than one property. For example, the flow data obtained by a flow sensor may be used to calculate physiological variables such as coronary flow reserve (CFR). The intraluminal device 102 includes a flexible elongate member 106. The sensor 113 is disposed at a distal portion 107 of the flexible elongate member 106. The sensor 113 may be mounted at the distal portion 107 within a housing 282 in some embodiments. A flexible tip coil 290 extends distally from the housing 282 at the distal portion 107 of the flexible elongate member 106. A connection portion 114 located at a proximal end of the flexible elongate member 106 includes conductive portions 132, 134. In some embodiments, the conductive portions 132, 134 may be conductive ink that is printed and/or deposited around the connection portion 114 of the flexible elongate member 106. In some embodiments, the conductive portions 132, 134 are conductive, may be metallic bands or rings that are positioned around the flexible elongate member. A locking area is formed by a collar or locking section 118 and knob or retention section 120 are disposed at the proximal portion 109 of the flexible elongate member 106.

The intraluminal device 102 in Fig. 2 includes core wire including a distal core 210 and a proximal core 220. The distal core 210 and the proximal core 220 are metallic components forming part of the body of the intraluminal device 102. For example, the distal core 210 and the proximal core 220 may be flexible metallic rods that provide structure for the flexible elongate member 106. The distal core 210 and/or the proximal core 220 may be made of a metal or metal alloy. For example, the distal core 210 and/or the proximal core 220 may be made of stainless steel, Nitinol, nickel-cobalt-chromium-molybdenum alloy (e.g., MP35N), and/or other suitable materials. In some embodiments, the distal core 210 and the proximal core 220 are made of the same material. In other embodiments, the distal core 210 and the proximal core 220 are made of different materials. The diameter of the distal core 210 and the proximal core 220 may vary along their respective lengths. A joint between the distal core 210 and proximal core 220 is surrounded and contained by a hypotube 215. The sensor 113 may in some cases be positioned at a distal end of the distal core 210.

In some embodiments, the intraluminal device 102 includes a distal subassembly and a proximal subassembly that are electrically and mechanically joined together, which creates an electrical communication between the sensor 113 and the conductive portions 132, 134. For example, flow data obtained by the sensor 113 (in this example, sensor 113 is a flow sensor) may be transmitted to the conductive portions 132, 134. In an exemplary embodiment, the sensor 113 is a single ultrasound transducer element. The transducer element emits ultrasound signals and receives echoes. The transducer element generates electrical signals representative of the echoes. The signal carrying filars carry this electrical signal from the sensor at the distal portion to the connector at the proximal portion. The processing system 306 processes the electrical signals to extract the flow velocity of the fluid.

Control signals from a processing system 306 (e.g., a processor circuit of the processing system 306) in communication with the intraluminal device 102 may be transmitted to the sensor 113 via a connector 314 that is attached to the conductive portions 132, 134. The distal subassembly may include the distal core 210. The distal subassembly may also include the sensor 113, the distal conductive members 230, and/or one or more layers of insulative polymer/plastic 240 surrounding the distal conductive members 230 and the distal core 210. For example, the polymer/plastic layer(s) may insulate and protect the conductive members of the multi-filar cable or conductor bundle. The proximal subassembly may include the proximal core 220. The proximal subassembly may also include one or more polymer layers 250 (hereinafter polymer layer 250) surrounding the proximal core 220 and/or conductive ribbons 260 embedded within the one or more insulative and/or protective polymer layer 250. In some embodiments, the proximal subassembly and the distal subassembly are separately manufactured. During the assembly process for the intraluminal device 102, the proximal subassembly and the distal subassembly may be electrically and mechanically joined together. As used herein, flexible elongate member may refer to one or more components along the entire length of the intraluminal device 102, one or more components of the proximal subassembly (e.g., including the proximal core 220, etc.), and/or one or more components the distal subassembly 410 (e.g., including the distal core 210, etc.). Accordingly, flexible elongate member may refer to the combined proximal and distal subassemblies described above. The joint between the proximal core 220 and distal core 210 is surrounded by the hypotube 215, which is a tubular member.

In various embodiments, the intraluminal device 102 may include one, two, three, or more core wires extending along its length. For example, a single core wire may extend substantially along the entire length of the flexible elongate member 106. In such embodiments, a locking section 118 and a retention section 120 may be integrally formed at the proximal portion of the single core wire. The sensor 113 may be secured at the distal portion of the single core wire. In other embodiments, such as the embodiment illustrated in Fig. 2, the locking section 118 and the retention section 120 may be integrally formed at the proximal portion of the proximal core 220. The sensor 113 may be secured at the distal portion of the distal core 210. The intraluminal device 102 includes one or more distal conductive members 230 (e.g., a multi-filar conductor bundle or cable) in communication with the sensor 113. For example, the distal conductive members 230 may be one or more electrical wires that are directly in communication with the sensor 113. In some instances, the distal conductive members 230 are electrically and mechanically coupled to and in electrical communication with the sensor 113 by, e.g., soldering. In some instances, the distal conductive members 230 includes two or three electrical wires (e.g., a bifilar cable or a trifilar cable). An individual electrical wire may include a bare metallic conductor surrounded by one or more insulating layers. The distal conductive members 230 may extend along the length of the distal core 210. For example, at least a portion of the distal conductive members 230 may be spirally wrapped around the distal core 210, minimizing or eliminating whipping of the distal core within tortuous anatomy.

The intraluminal device 102 includes one or more conductive ribbons 260 at the proximal portion of the flexible elongate member 106. The conductive ribbons 260 are embedded within polymer layer 250. The conductive ribbons 260 are directly in communication with the conductive portions 132 and/or 134. In some instances, the distal conductive members 230 are electrically and mechanically coupled to and in electrical communication with the sensor 113 by, e.g., soldering. In some instances, the conductive portions 132 and/or 134 includes conductive ink (e.g., metallic nano-ink, such as copper, silver, gold, or aluminum nano-ink) that is deposited or printed directed over the conductive ribbons 260.

As described herein, electrical communication between the distal conductive members 230 and the conductive ribbons 260 may be established at the connection portion 114 of the flexible elongate member 106. By establishing electrical communication between the distal conductive members 230 and the conductive ribbons 260, the conductive portions 132, 134 may be in electrical communication with the sensor 113.

In some embodiments represented by Fig. 1, the intraluminal device 102 includes a locking section 118 and knob or retention section 120. To form locking section 118, a machining process is used to remove polymer layer 250 and conductive ribbons 260 in locking section 118 and to shape proximal core 220 in locking section 118 to the desired shape. As shown in Fig. 1, locking section 118 includes a reduced diameter while knob or retention has a diameter substantially similar to that of proximal core 220 in the connection portion 114. In some instances, because the machining process removes conductive ribbons in locking section 118, proximal ends of the conductive ribbons 260 would be exposed to moisture and/or liquids, such as blood, saline solutions, disinfectants, and/or enzyme cleaner solutions, an insulation layer 158 is formed over the proximal end portion of the connection portion 114 to insulate the exposed conductive ribbons 260.

In some embodiments, a connector 314 provides electrical connectivity between the conductive portions 132, 134 and a patient interface monitor 304. The Patient Interface Monitor (PIM) 304 may in some cases connect to a console or processing system 306, which includes or is in communication with a display 308.

The intraluminal sensing system 100 may be deployed in a catheterization laboratory having a control room. The processing system 306 may be located in the control room. Optionally, the processing system 306 may be located elsewhere, such as in the catheterization laboratory itself. The catheterization laboratory may include a sterile field while its associated control room may or may not be sterile depending on the procedure to be performed and/or on the health care facility. In some embodiments, the intraluminal device 102 may be controlled from a remote location such as the control room, such that an operator is not required to be in close proximity to the patient.

The intraluminal device 102, PIM 304, and display 308 may be communicatively coupled directly or indirectly to the processing system 306. These elements may be communicatively coupled to the processing system 306 via a wired connection such as a standard copper multi-filar conductor bundle. The processing system 306 may be communicatively coupled to one or more data networks, e.g., a TCP/IP-based local area network (LAN). In other embodiments, different protocols may be utilized such as Synchronous Optical Networking (SONET). In some cases, the processing system 306 may be communicatively coupled to a wide area network (WAN).

The PIM 304 transfers the received signals to the processing system 306 where the information is processed and displayed (e.g., as physiology data in graphical, symbolic, or alphanumeric form) on the display 308. The console or processing system 306 may include a processor and a memory. The processing system 306 may be operable to facilitate the features of the intraluminal sensing system 100 described herein. For example, the processor may execute computer readable instructions stored on the non-transitory tangible computer readable medium.

The PIM 304 facilitates communication of signals between the processing system 306 and the intraluminal device 102. The PIM 304 may be communicatively positioned between the processing system 306 and the intraluminal device 102. In some embodiments, the PIM 304 performs preliminary processing of data prior to relaying the data to the processing system 306. In examples of such embodiments, the PIM 304 performs amplification, filtering, and/or aggregating of the data. In an embodiment, the PIM 304 also supplies high- and low-voltage DC power to support operation of the intraluminal device 102 via the distal conductive members 230.

A multi-filar cable or transmission line bundle, such as distal conductive members 230, may include a plurality of conductors, including one, two, three, four, five, six, seven, or more conductors. In the example shown in Fig. 2, the multi-filar conductor bundle includes two straight portions 232 and 236, where the multi-filar conductor bundle lies parallel to a longitudinal axis of the flexible elongate member 106, and a spiral portion 234, where the multi-filar conductor bundle is wrapped around the exterior of the flexible elongate member 106 and then overcoated with an insulative and/or protective polymer 240. Communication, if any, along the multi-filar conductor bundle may be through numerous methods or protocols, including serial, parallel, and otherwise, where one or more filars of the of the multi-filar conductor bundle carry signals. One or more filars of the multi-filar conductor bundle may also carry direct current (DC) power, alternating current (AC) power, or serve as a ground connection.

The display or monitor 308 may be a display device such as a computer monitor or other type of screen. The display or monitor 308 may be used to display selectable prompts, instructions, and visualizations of imaging data to a user. In some embodiments, the display 308 may be used to provide a procedure-specific workflow to a user to complete an intraluminal imaging procedure.

Before continuing, it should be noted that the examples described above are provided for purposes of illustration and are not intended to be limiting. Other devices and/or device configurations may be utilized to carry out the operations described herein.

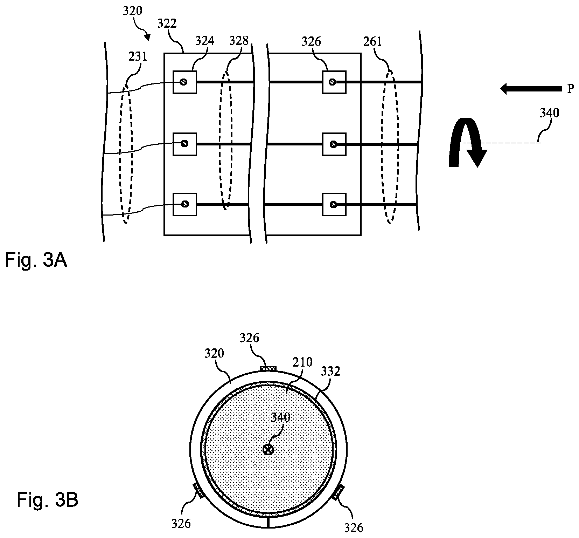

Fig. 3A is a schematic top view of an unrolled flex circuit 320 for application to a portion of the intraluminal device 102 with electrical conductors/traces 328 formed in the flex circuit 320, according to aspects of the present disclosure. In some embodiments, the flex circuit 320 may be formed in a separate process before being applied to a distal core 210. The flex circuit 320 is a thin film that includes a flexible substrate 322 onto or within which a plurality of electrical conductors/traces 328 are formed. In some embodiments, the flex circuit 320 may be formed by disposing the electrical conductors/traces 328 onto a polymer base and then overcoating the electrical conductors/traces 328 with a polymer topcoat.

The flexible substrate 322 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 3A, the flexible substrate 322 has a generally rectangular shape. As shown and described herein, the flexible substrate 322 is configured to be wrapped partially or completely around an exterior the distal core 210 in some instances. Therefore, the thickness of the film layer of the flexible substrate 322 is generally related to the degree of curvature in the final assembled flexible elongate member 106. In some embodiments, the film layer is between 5 µm and 100 µm, with some particular embodiments being between 5 µm and 25.1 µm, e.g., 6 µm.

To electrically interconnect the conductive members 231 and the conductive member 261, in an embodiment, the flexible substrate 322 includes conductive traces 328 formed in the film layer that carry signals between the conductive members 231 and the conductive members 261. In particular, the conductive traces 328 provide communication between the conductive members 231 and the conductive members 261 extending along the flexible substrate 322. In some instances, the conductive traces 328 may also facilitate electrical communication between the PIM 304 and the sensor 112/113. The conductive traces 328 may also provide a set of conductive pads 324 and 326 that contact the conductive members 231 and the conductive members 261, respectively, when the conductive members 231 and the conductive members 261 are mechanically and electrically coupled to the flexible circuit 320. Suitable materials for the conductive traces 328 include copper, gold, aluminum, silver, tantalum, nickel, and tin, and may be deposited on the flexible substrate 322 by processes such as sputtering, plating, and etching. In an embodiment, the flexible substrate 322 includes a chromium adhesion layer. The width and thickness of the conductive traces 328 are selected to provide proper conductivity and resilience when the flexible substrate 322 is rolled. In that regard, an exemplary range for the thickness of a conductive traces 328 and/or conductive pad is between 1-5 µm. For example, in an embodiment, 5 µm conductive traces 328 are separated by 5 µm of space. The width of a conductive trace 328 on the flexible substrate may be further determined by the width of the conductive members 231 and the conductive members 261 to be coupled to the trace/pad.

In some embodiments, the electrical conductors/traces 328 are distinct electrical traces in that they are physically and electrically separate from one another, but structurally made of a same material. In other embodiments, the electrical conductors/traces 328 may structurally be made of different materials. On a distal end of the flexible substrate 322, a plurality of distal conductive pads 324 are formed and are electrically and mechanically coupled to respective ones of the plurality of electrical conductors/traces 328 by, e.g., soldering, ultrasonic welding, conductive adhesive, etc. Additionally, on a proximal end of the flexible substrate 322, a plurality of proximal conductive pads 326 are formed and are electrically and mechanically coupled to respective ones of the plurality of electrical conductors/traces 328 by, e.g., soldering, ultrasonic welding, conductive adhesive, etc.

In some embodiments, the flex circuit 320 may be in the form of a sheet that is wrapped around an exterior of the distal core 210 along a longitudinal axis such that edges of the flex circuit 320 meet. That is, the flex circuit 320 extends parallel to a longitudinal axis of the flexible elongate member 106 over the distal core 210. In some embodiments, the flex circuit 320 may be in the form of a strip such that the strip is spirally wrapped around the distal core 210. Once applied to the distal core 210, electrical connections are made to conductive members 231 and conductive members 261. In some embodiments, conductive members 231 and conductive members 261 may be multi-filar cables. In some embodiments, conductive members 261 may be conductive ribbons, such as conductive ribbons 260. In some embodiments, respective ones of conductive members 231 are electrically and mechanically coupled to respective ones of the plurality of distal conductive pads 324 by, e.g., soldering, ultrasonic welding, conductive adhesive, etc. In some embodiments, respective ones of conductive members 261 are electrically and mechanically coupled to respective ones of the plurality of proximal conductive pads 326 by, e.g., soldering, ultrasonic welding, conductive adhesive, etc. In some embodiments, respective ones of conductive ribbons 260 are directly coupled to the respective ones of the plurality of proximal conductive pads 326. That is, conductive ribbons 260 may be formed past the connection region 270 and the polymer layer 250 in which the conductive ribbons 260 are embedded is removed such the conductive ribbons 260 may be directly coupled to respective ones of the plurality of proximal conductive pads 326. In some embodiments, respective ones of conductive ribbons 260 may be indirectly coupled to the respective ones of the plurality of proximal conductive pads 326. That is, respective distal ends of the conductive members 261 may be coupled to respective ones of the plurality of proximal conductive pads 326 and respective proximal ends of the conductive members 261 may be coupled to respective ones of the conductive ribbons 260 the connection region 270.

The intraluminal device 102 may include any suitable quantity of conductors in the flex circuit 320, e.g., two, three, four, five, or more. In some embodiments, the intraluminal device 102 includes the same quantity of conductors in the flex circuit 320 as the distal conductive members 230. In some embodiments, a communication line is established between one of conductive members 231, electrical conductors/traces 328, and one of conductive members 261 that are electrically coupled/in communication with one another. In some embodiments, there are two or more communication lines for, e.g., power (positive, negative, ground), signal/data transmission.

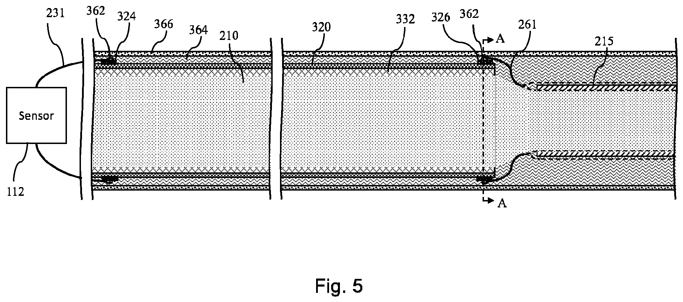

Fig. 3B is a schematic end view of a rolled flex circuit 320 for application to a portion of the intraluminal device 102 from direction P shown in Fig. 3A rolled in a cylindrical configuration along longitudinal axis 340 and a cross-sectional end view along section line A-A in Fig. 5, according to aspects of the present disclosure. Fig. 3B is not a complete cross-sectional end view of Fig. 5, because Fig. 3 does not include all of components in the section of Fig. 5. That is, Fig. 3B shows a subset of the components including distal core 210, adhesive 332, flex circuit 320, and proximal conductive pads 326, Fig. 5 shows that subset and additionally shows polymer coating 364 and hydrophilic coating 366. In the depicted embodiment illustrated in Fig. 3B, the flex circuit 320 is wrapped around, in this instance, the distal core 210. Flex circuit 320 surrounds and couples to the distal core 210 via adhesive 332. That is, adhesive 332 is in contact with the distal core 210 and flex circuit 320 is in contact with adhesive 332. Also visible from direction P, are the plurality of proximal conductive pads 326.

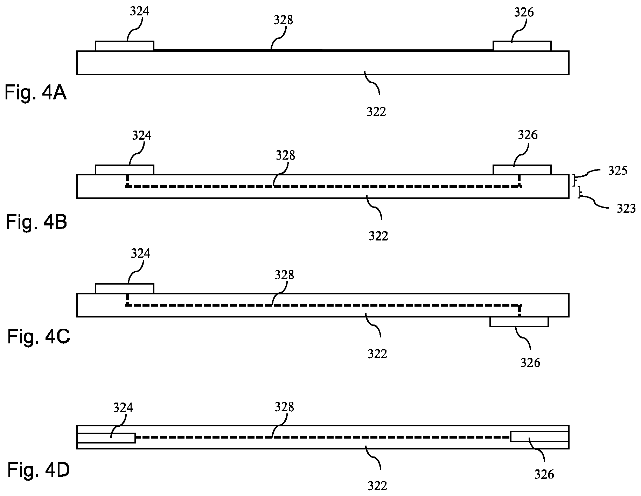

Fig. 4A is a schematic side view of a portion of the flex circuit 320 including the electrical conductors/traces 328 disposed onto the flexible substrate 322 of the flex circuit 320, according to aspects of the present disclosure. That is, the flex circuit 320 is a thin film that includes the flexible substrate 322 onto which, in this illustration, the electrical conductors/traces 328 are disposed. On a distal end of the flexible substrate 322, a distal conductive pad 324 is formed. On a proximal end of the flexible substrate 322, a proximal conductive pad 326 is formed on a same side of the flexible substrate 322 that the distal conductive pad 324 is formed.

Fig. 4B is a schematic side view of a portion of the flex circuit 320 including the electrical conductors/traces 328 disposed within the flexible substrate 322 of the flex circuit 320, according to aspects of the present disclosure. In this illustration, the electrical conductors/traces 328 are formed within the substrate by, for example, forming a portion of the substrate (e.g., a polymer base 323), disposing the electrical conductors/traces 328 (e.g., on the polymer base 323), and then forming the rest of the flexible substrate 322 over the electrical conductors/traces 328 (e.g., the polymer topcoat 325). Proximal conductive pad 326 may be formed over the polymer coating 364. In this illustration, the distal conductive pad 324 and the proximal conductive pad 326 are formed on a same side of the flexible substrate 322.

Fig. 4C is a schematic side view of a portion of the flex circuit 320 including the electrical conductors/traces 328 disposed within the flexible substrate 322 of the flex circuit 320, according to aspects of the present disclosure. In this illustration, the distal conductive pad 324 and the proximal conductive pad 326 are formed on opposite sides of the flexible substrate 322.

Fig. 4D is a schematic side view of a portion of the flex circuit 320 including the electrical conductors/traces 328 disposed within the flexible substrate 322 of the flex circuit 320, according to aspects of the present disclosure. In this illustration, the distal conductive pad 324 and the proximal conductive pad 326 are formed in the ends of the flexible substrate 322.

Fig. 5 is a diagrammatic side view of a portion of an intraluminal device 102 with electrical conductors/traces 328 formed in the flex circuit 320, according to aspects of the present disclosure. Visible is the flex circuit 320 is joined to the distal core 210 by adhesive 332. That is, adhesive 332 is in contact the distal core 210 and flex circuit 320 is in contact with the adhesive 332. While Fig. 5 illustrates adhesive 332 along the entire length of the wider portion of distal core 210, in some embodiments, adhesive 332 may be used only at the ends of the flex circuit 320. In some embodiments, adhesive 332 may be spaced apart, such as every 3cm, 5cm, etc. The flex circuit 320 is a thin film that includes a flexible substrate 322 onto or within which a plurality of electrical conductors/traces 328 are formed. In embodiments where adhesive 332 is not used along the entire length of the wider portion of distal core 210, the flexible substrate 322 of the flex circuit 320 may directly contact the distal core 210.

The flex circuit 320 may be positioned around a majority of the length of the distal core. In some embodiments, the distal core 210 may be between 30 cm and 50 cm, e.g. 40 cm. In some embodiments, the proximal core 220 may be between 130 cm and 160 cm, e.g. 145 cm. In some embodiments, the flex circuit 320 may be between 20 cm and 60 cm, between 20 cm and 40 cm, etc., e.g. 30-40 cm. Therefore, for example, the flex circuit 320 may be between 50% and 100% of the length of the distal core 210, between 66% and 100% of the length of the distal core 210, between 75% and 100% of the length of the distal core 210, etc. In some embodiments, the length of the flex circuit 320 is less than the length of the distal core 210. In some embodiments, the length of the flex circuit 320 is equal to length of distal core 210. In some embodiments, the length of the flex circuit 320 may be longer than the length of the distal core 210. The flex circuit 320 may be longitudinally centered relative to the distal core 210. The flex circuit 320 may be positioned closer to the distal end of the distal core 210 (e.g., distal ends of distal core 210 and flex circuit 320 are aligned or spaced apart from one another). The flex circuit 320 may be positioned closer to the proximal end of the distal core 210 (e.g.., proximal ends of distal core 210 and flex circuit 320 are aligned or spaced apart from one another). In some embodiments, all of the length of the flex circuit 320 is positioned around the distal core 210. In some embodiments, a portion of the flex circuit 320 may not be positioned around the distal core 210. For example, a distal end of the flex circuit 320 may be distal of the distal end of the distal core 210. For example, a proximal end of the flex circuit 320 may be proximal of the proximal end of the distal core 210.

In some instances, on a distal end of the flex circuit 320, a plurality of distal conductive pads 324 are formed and are electrically and mechanically coupled to respective ones of the plurality of electrical conductors/traces 328 by, e.g., soldering, ultrasonic welding, conductive adhesive, etc. In that regard, respective proximal ends of conductive members 231 are coupled to respective ones of the plurality of distal conductive pads 324 by a coupling 362, e.g., soldering, ultrasonic welding, conductive adhesive, etc. Respective distal ends of conductive members 231 are coupled to respective pads of sensor 112 by, e.g., soldering, ultrasonic welding, conductive adhesive, etc.

In some instances, the flex circuit 320 directly couples (e.g., mechanically and/or electrically connects) to the sensor 112. For example, a distal portion of the flex circuit 320 can overlap along a length of the intraluminal device with a portion of the sensor 112 including conductive pads. In some instances, the distal portion of the flex circuit 320 (with conductive pads 324) is positioned over the proximal portion of the sensor 112 (with conductive pads of the sensor 112). The conductive pads of the sensor 112 receive electrical signals generated by the sensor 112 (e.g., a pressure-sensing diaphragm, a flow-sensing ultrasound transducer, etc.) Conductive pads 324 of the flex circuit 320 (at the distal portion of the flex circuit 320) can directly couple (e.g., mechanically and/or electrically connect) to conductive pads of the sensor 112. Accordingly, the sensor 112 is in electrical communication with the plurality of electrical conductors/traces 328 of the flex circuit 320 via the coupling between the conductive pads of the flex circuit 320 and the conductive pads 324 of the flex circuit. In such instances, conductive members 231 are omitted so that there is no intermediate conductive member between the flex circuit 320 and the sensor 112.

On a proximal end of the flex circuit 320, a plurality of proximal conductive pads 326 are formed and are electrically and mechanically coupled to respective ones of the plurality of electrical conductors/traces 328. Respective ones of conductive members 261 are coupled to the respective ones of the plurality of proximal conductive pads 326 by a coupling 362, e.g., soldering, ultrasonic welding, conductive adhesive, etc. In some embodiments, conductive members 261 may couple to conductive ribbons 260 (also referred to as proximal conductive members 260) associated with the proximal core 220 via a multi-filar cable that runs on an exterior or an interior of hypotube 215. A polymer coating 364 is then applied, which is then overcoated with hydrophilic coating 366.

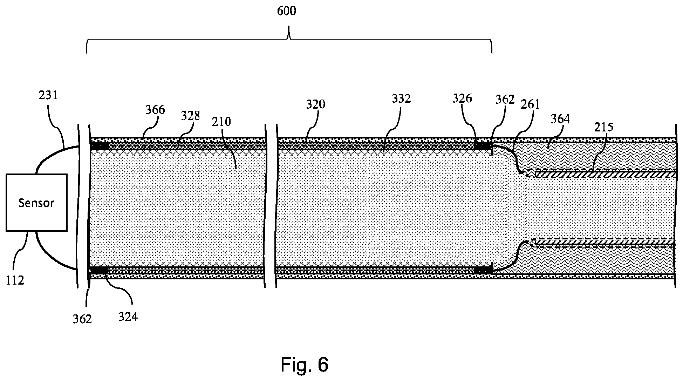

Fig. 6 is a diagrammatic cross-sectional side view of a portion of an intraluminal device 102 with electrical conductors/traces 328 formed in the flex circuit 320, according to aspects of the present disclosure. Fig. 6 includes features similar to those described in Fig. 5. In the embodiment of Fig. 6, in section 600, the flex circuit 320 replaces the polymer coating 364. In some embodiments, replacing the polymer coating 364 in section 600 with the flex circuit 320 allows for an increased diameter of distal core 210. In order for flex circuit 320 to replace the polymer coating 364, the distal conductive pad 324 and the proximal conductive pad 326 are formed in the ends of the flexible substrate 322. As with Fig. 5, in Fig. 6 the flex circuit 320 is joined to the distal core 210 by adhesive 332. That is, adhesive 332 is in contact the distal core 210 and flex circuit 320 is in contact with the adhesive 332. While Fig. 6 illustrates adhesive 332 along the entire length of the wider portion of distal core 210, in some embodiments, adhesive 332 may be used only at the ends of the flex circuit 320. In some embodiments, adhesive 332 may be spaced apart, such as every 3cm, 5cm, etc. In embodiments where adhesive 332 is not used along the entire length of the wider portion of distal core 210, the flexible substrate 322 of the flex circuit 320 may directly contact the distal core 210. The flex circuit 320 is then overcoated with hydrophilic coating 366.

In some instances, the flex circuit 320 directly couples (e.g., mechanically and/or electrically connects) to the sensor 112. For example, a distal portion of the flex circuit 320 can overlap along a length of the intraluminal device with a portion of the sensor 112 including conductive pads. In some instances, the distal portion of the flex circuit 320 (with conductive pads 324) is positioned over the proximal portion of the sensor 112 (with conductive pads of the sensor 112). The conductive pads of the sensor 112 receive electrical signals generated by the sensor 112 (e.g., a pressure-sensing diaphragm, a flow-sensing ultrasound transducer, etc.) Conductive pads 324 of the flex circuit 320 (at the distal portion of the flex circuit 320) can directly couple (e.g., mechanically and/or electrically connect) to conductive pads of the sensor 112. Accordingly, the sensor 112 is in electrical communication with the plurality of electrical conductors/traces 328 of the flex circuit 320 via the coupling between the conductive pads of the flex circuit 320 and the conductive pads 324 of the flex circuit. In such instances, conductive members 231 are omitted so that there is no intermediate conductive member between the flex circuit 320 and the sensor 112.

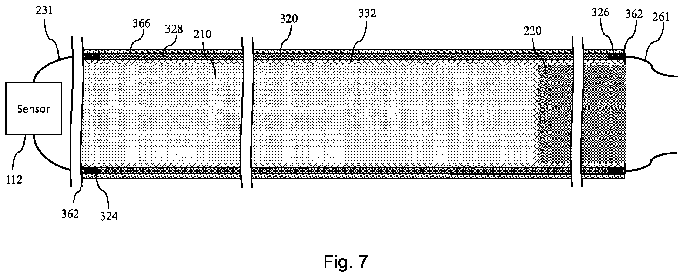

Fig. 7 is a diagrammatic cross-sectional side view of a portion of an intraluminal device 102 with electrical conductors/traces 328 formed in the flex circuit 320, according to aspects of the present disclosure. Fig. 7 includes features similar to those described in Fig. 6. In the embodiment in Fig. 7, the flex circuit 320 is applied along a length of the distal core 210 and the proximal core 220, replacing not only the polymer coating 364 but also the hypotube 215. In some embodiments, replacing the polymer coating 364 along a length of the distal core 210 and the proximal core 220 allows for an increased diameter of not only the distal core 210 but also the proximal core 220. In order for flex circuit 320 to replace the polymer coating 364, the distal conductive pad 324 and the proximal conductive pad 326 are formed in the ends of the flexible substrate 322. In some embodiments, the flex circuit 320 is joined to the distal core 210 and the proximal core 220 by adhesive 332. That is, adhesive 332 is in contact the distal core 210 and the proximal core 220 and the flex circuit 320 is in contact with the adhesive 332. While Fig. 7 illustrates adhesive 332 along the entire length of the distal core 210 and the proximal core 220, in some embodiments, adhesive 332 may be used only at the ends of the flex circuit 320. In some embodiments, adhesive 332 may be spaced apart, such as every 3cm, 5cm, etc. In embodiments where adhesive 332 is not used along the entire length of the wider portion of distal core 210, the flexible substrate 322 of the flex circuit 320 may directly contact the distal core 210. The flex circuit 320 is then overcoated with hydrophilic coating 366.

In some instances, the flex circuit 320 directly couples (e.g., mechanically and/or electrically connects) to the sensor 112. For example, a distal portion of the flex circuit 320 can overlap along a length of the intraluminal device with a portion of the sensor 112 including conductive pads. In some instances, the distal portion of the flex circuit 320 (with conductive pads 324) is positioned over the proximal portion of the sensor 112 (with conductive pads of the sensor 112). The conductive pads of the sensor 112 receive electrical signals generated by the sensor 112 (e.g., a pressure-sensing diaphragm, a flow-sensing ultrasound transducer, etc.) Conductive pads 324 of the flex circuit 320 (at the distal portion of the flex circuit 320) can directly couple (e.g., mechanically and/or electrically connect) to conductive pads of the sensor 112. Accordingly, the sensor 112 is in electrical communication with the plurality of electrical conductors/traces 328 of the flex circuit 320 via the coupling between the conductive pads of the flex circuit 320 and the conductive pads 324 of the flex circuit. In such instances, conductive members 231 are omitted so that there is no intermediate conductive member between the flex circuit 320 and the sensor 112.

Accordingly, it may be seen that the flex circuit assembly advantageously enables the use of conductive materials that may be formed separately and then applied to one or components of a flexible elongate member. Additionally, flex circuits allow for a larger outside diameter (OD) of the core wires and eliminate damage/scrap due to multi-filar bundle handling and thus, may enable lower electrical resistance/impedance along the length of the electro-mechanical intraluminal sensing device. A straightness and torque response of the intraluminal device may also be improved because ribbons and/or multi-filar conductor bundles, which would be reduced or eliminated, add stiffness and local torques to a guidewire or catheter. A number of variations are possible on the examples and embodiments described above. The flex circuit assembly could be applied to any product that involves electrical conductors to be embedded into composite subassemblies. However, unlike conductive ribbons, flex circuits may conform to the curvature of the core wire.

The logical operations making up the embodiments of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the metal ink conductor assembly. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the metal ink conductor assembly as defined in the claims. Although various embodiments of the claimed subject matter have been described above with a certain degree of particularity, or with reference to one or more individual embodiments, those skilled in the art could make numerous alterations to the disclosed embodiments without departing from the scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the scope of the invention as defined in the following claims.

## Claims

1. An intraluminal device (102), comprising:
a flexible elongate member (106) configured to be positioned within a body lumen of a patient, wherein the flexible elongate member comprises:
a distal portion (107) comprising a distal core member (210) and a sensor (112), wherein the distal core member comprises a length between a distal end and a proximal end;
a proximal portion (109) comprising a proximal core member (220), a polymer layer surrounding the proximal core member and a proximal electrical conductor (261, 260), the proximal electrical conductor comprising conductive ribbons (260) embedded within the polymer layer; and
a flex circuit (320) positioned around a majority of the length of the distal core member (210), wherein all of the length of the flex circuit is positioned around the distal core member, wherein the flex circuit comprises a distal portion coupled to the sensor (112) and a proximal portion coupled to the proximal electrical conductor (261, 260) such that the proximal electrical conductor is in electrical communication with the sensor (112) via the flex circuit, wherein the sensor is disposed distally from the distal portion of the flex circuit.

2. The intraluminal device of claim 1, wherein the flex circuit comprises:
a polymer base (322); and
a conductive trace (328) formed on the polymer base.

3. The intraluminal device of claim 2, wherein the flex circuit further comprises a polymer topcoat formed over the conductive trace (328).

4. The intraluminal device of claim 2,
wherein the flex circuit further comprises:
a distal conductive pad (324) formed on the polymer base at a distal portion of the flex circuit; and
a proximal conductive pad (326) formed on the polymer base at a proximal portion of flex circuit,
wherein the conductive trace (328) is coupled to the distal conductive pad and the proximal conductive pad,
wherein the proximal electrical conductor is coupled to the flex circuit at the proximal conductive pad, and
wherein the sensor is coupled to the flex circuit at the distal conductive pad.

5. The intraluminal device of claim 1, further comprising an adhesive (332) positioned between the flex circuit and the distal core member (210), wherein the adhesive is configured to couple the flex circuit and the distal core member.

6. The intraluminal device of claim 1, wherein the flex circuit is wrapped in a spiral pattern around the distal core member.

7. The intraluminal device of claim 1, wherein the flex circuit is rolled in a cylindrical configuration around the distal core member.

8. The intraluminal device of claim 1, wherein the flex circuit is formed prior to being positioned around the distal core member, wherein the flex circuit is formed as a sheet, wherein at least one conductive trace of the flex circuit is formed across the sheet, and wherein the sheet is wrapped around the distal core member.

9. The intraluminal device of claim 1, wherein the distal portion further comprises:
a polymer coating positioned around the flex circuit.

10. The intraluminal device of claim 9, wherein the polymer coating is in direct contact with the flex circuit.

11. The intraluminal device of claim 1, wherein the distal portion further comprises:
a hydrophilic coating positioned around the flex circuit.

12. The intraluminal device of claim 11, wherein the hydrophilic coating is in direct contact with the flex circuit.

13. The intraluminal device of claim 1, wherein the flexible elongate member is an intravascular guidewire and the body lumen is a blood vessel.

14. An intraluminal sensing system comprising an intraluminal device of any one of claims 1 to 13.

## Patentansprüche

1. Intraluminale Vorrichtung (102), die Folgendes umfasst:
ein flexibles längliches Element (106), das dazu ausgelegt ist, innerhalb eines Körperlumens eines Patienten positioniert zu werden, wobei das flexible längliche Element Folgendes umfasst:
einen distalen Abschnitt (107), der ein distales Kernelement (210) und einen Sensor (112) umfasst, wobei das distale Kernelement eine Länge zwischen einem distalen Ende und einem proximalen Ende umfasst;
einen proximalen Abschnitt (109), der ein proximales Kernelement (220), eine Polymerschicht, die das proximale Kernelement umgibt, und einen proximalen elektrischen Leiter (261, 260) umfasst, wobei der proximale elektrische Leiter leitfähige Bänder (260) umfasst, die in die Polymerschicht eingebettet sind; und
eine flexible Schaltung (320), die um einen Großteil der Länge des distalen Kernelements (210) positioniert ist,
wobei die gesamte Länge der flexiblen Schaltung um das distale Kernelement positioniert ist, wobei die flexible Schaltung einen distalen Abschnitt, der mit dem Sensor (112) gekoppelt ist, und einen proximalen Abschnitt, der mit dem proximalen elektrischen Leiter (261, 260) gekoppelt ist, umfasst, sodass der proximale elektrische Leiter über die flexible Schaltung in elektrischer Kommunikation mit dem Sensor (112) steht, wobei der Sensor distal von dem distalen Abschnitt der flexiblen Schaltung angeordnet ist.

2. Intraluminale Vorrichtung nach Anspruch 1, wobei die flexible Schaltung Folgendes umfasst:
eine Polymerbasis (322); und
eine Leiterbahn (328), die auf der Polymerbasis gebildet ist.

3. Intraluminale Vorrichtung nach Anspruch 2, wobei die flexible Schaltung ferner eine Polymerdeckbeschichtung umfasst, die über der Leiterbahn (328) gebildet ist.

4. Intraluminale Vorrichtung nach Anspruch 2,
wobei die flexible Schaltung ferner Folgendes umfasst:
ein distales leitfähiges Pad (324), das auf der Polymerbasis an einem distalen Abschnitt der flexiblen Schaltung gebildet ist; und
ein proximales leitfähiges Pad (326), das auf der Polymerbasis an einem proximalen Abschnitt der flexiblen Schaltung gebildet ist,
wobei die Leiterbahn (328) mit dem distalen leitfähigen Pad und dem proximalen leitfähigen Pad gekoppelt ist,
wobei der proximale elektrische Leiter an dem proximalen leitfähigen Pad mit der flexiblen Schaltung gekoppelt ist, und
wobei der Sensor an dem distalen leitfähigen Pad mit der flexiblen Schaltung gekoppelt ist.

5. Intraluminale Vorrichtung nach Anspruch 1, die ferner einen Klebstoff (332) umfasst, der zwischen der flexiblen Schaltung und dem distalen Kernelement (210) positioniert ist, wobei der Klebstoff dazu ausgelegt ist, die flexible Schaltung und das distale Kernelement zu koppeln.

6. Intraluminale Vorrichtung nach Anspruch 1, wobei die flexible Schaltung in einem Spiralmuster um das distale Kernelement gewickelt ist.

7. Intraluminale Vorrichtung nach Anspruch 1, wobei die flexible Schaltung in einer zylindrischen Auslegung um das distale Kernelement gerollt ist.

8. Intraluminale Vorrichtung nach Anspruch 1, wobei die flexible Schaltung gebildet wird, bevor sie um das distale Kernelement herum positioniert wird, wobei die flexible Schaltung als eine Folie ausgebildet ist, wobei mindestens eine Leiterbahn der flexiblen Schaltung über die ganze Folie gebildet wird und wobei die Folie um das distale Kernelement herum gewickelt ist.

9. Intraluminale Vorrichtung nach Anspruch 1, wobei der distale Abschnitt ferner Folgendes umfasst:
eine Polymerbeschichtung, die um die flexible Schaltung herum positioniert ist.

10. Intraluminale Vorrichtung nach Anspruch 9, wobei sich die Polymerbeschichtung in direktem Kontakt mit der flexiblen Schaltung befindet.

11. Intraluminale Vorrichtung nach Anspruch 1, wobei der distale Abschnitt ferner Folgendes umfasst:
eine hydrophile Beschichtung, die um die flexible Schaltung herum angeordnet ist.

12. Intraluminale Vorrichtung nach Anspruch 11, wobei die hydrophile Beschichtung in direktem Kontakt mit der flexiblen Schaltung steht.

13. Intraluminale Vorrichtung nach Anspruch 1, wobei das flexible längliche Element ein intravaskulärer Führungsdraht ist und das Körperlumen ein Blutgefäß ist.

14. Intraluminales Erfassungssystem, das eine intraluminale Vorrichtung nach einem der Ansprüche 1 bis 13 umfasst.

## Revendications

1. Dispositif intraluminal (102) comprenant :
un élément allongé flexible (106) configuré pour être positionné à l'intérieur d'une lumière corporelle d'un patient, l'élément allongé flexible comprenant :
une partie distale (107) comprenant un élément de noyau distal (210) et un capteur (112), l'élément de noyau distal comprenant une longueur entre une extrémité distale et une extrémité proximale ;
une partie proximale (109) comprenant un élément de noyau proximal (220), une couche polymère entourant l'élément de noyau proximal et un conducteur électrique proximal (261, 260), le conducteur électrique proximal comprenant des rubans conducteurs (260) noyés dans la couche polymère ; et
un circuit flexible (320) positionné autour de la majorité de la longueur de l'élément de noyau distal (210), toute la longueur du circuit flexible étant positionnée autour de l'élément de noyau distal, le circuit flexible comprenant une partie distale couplée au capteur (112) et une partie proximale couplée au conducteur électrique proximal (261, 260) de telle sorte que le conducteur électrique proximal est en communication électrique avec le capteur (112) via le circuit flexible, le capteur étant disposé distalement par rapport à la partie distale du circuit flexible.

2. Dispositif intraluminal selon la revendication 1, le circuit flexible comprenant :
une base polymère (322) ; et
une trace conductrice (328) formée sur la base polymère.

3. Dispositif intraluminal selon la revendication 2, le circuit flexible comprenant en outre une couche de finition polymère formée sur la trace conductrice (328).

4. Dispositif intraluminal selon la revendication 2, le circuit flexible comprenant en outre :
un plot conducteur distal (324) formé sur la base polymère au niveau d'une partie distale du circuit flexible ; et
un plot conducteur proximal (326) formé sur la base polymère au niveau d'une partie proximale du circuit flexible,
la trace conductrice (328) étant couplée au plot conducteur distal et au plot conducteur proximal,
le conducteur électrique proximal étant couplé au circuit flexible au niveau du plot conducteur proximal, et
le capteur étant couplé au circuit flexible au niveau du plot conducteur distal.

5. Dispositif intraluminal selon la revendication 1, comprenant en outre un adhésif (332) positionné entre le circuit flexible et l'élément de noyau distal (210), l'adhésif étant configuré pour coupler le circuit flexible et l'élément de noyau distal.

6. Dispositif intraluminal selon la revendication 1, le circuit flexible étant enroulé selon un motif en spirale autour de l'élément de noyau distal.

7. Dispositif intraluminal selon la revendication 1, le circuit flexible étant enroulé dans une configuration cylindrique autour de l'élément de noyau distal.

8. Dispositif intraluminal selon la revendication 1, le circuit flexible étant formé avant d'être positionné autour de l'élément de noyau distal, le circuit flexible étant formé sous la forme d'une feuille, au moins une trace conductrice du circuit flexible étant formée à travers la feuille, et la feuille étant enroulée autour de l'élément de noyau distal.

9. Dispositif intraluminal selon la revendication 1, la partie distale comprenant en outre :
un revêtement polymère positionné autour du circuit flexible.

10. Dispositif intraluminal selon la revendication 9, le revêtement polymère étant en contact direct avec le circuit flexible.

11. Dispositif intraluminal selon la revendication 1, la partie distale comprenant en outre :
un revêtement hydrophile positionné autour du circuit flexible.

12. Dispositif intraluminal selon la revendication 11, le revêtement hydrophile étant en contact direct avec le circuit flexible.

13. Dispositif intraluminal selon la revendication 1, l'élément allongé flexible étant un fil-guide intravasculaire et la lumière corporelle étant un vaisseau sanguin.

14. Système de détection intraluminal comprenant un dispositif intraluminal selon l'une quelconque des revendications 1 à 13.
